Europäisches Patentamt

European Patent Office

Office européen des brevets

(19)

(11) Publication number: **0 495 443 A1**

## EUROPEAN PATENT APPLICATION

(12)

(21) Application number: **92100477.6**

(22) Date of filing: **14.01.92**

(51) Int. Cl.5: **A61N 5/06**, A61F 7/12,
A61B 17/36

(30) Priority: **14.01.91 CN 91100301**
**09.01.92 CN 92100043**

(43) Date of publication of application:
**22.07.92 Bulletin 92/30**

(84) Designated Contracting States:
**DE FR GB IT**

(71) Applicant: **DALIAN POME INSTRUMENT
EOUIPMENT CORP. Ltd.
Room 101, 122 Xicun Heishijiao
Shakekou District, Dalian, Liaoning
Province(CN)**

(72) Inventor: **Chang, Ruiming Room 101, 122
Xicun Heishijiao
Shahekou District Dalain
Liaoning Province(CN)**
Inventor: **Hao, Peiming Room 101, 122 Xicun
Heishijiao
Shahekou District Dalian
Liaoning Province(CN)**

(74) Representative: **Rau, Manfred, Dr. Dipl.-Ing. et
al
Rau & Schneck, Patentanwälte Königstrasse
2
W-8500 Nürnberg 1(DE)**

(54) Optical radiation treatment instrument.

(57) An optical radiation treatment instrument, especially an optical radiation treatment instrument employing the heat effect generated by radiation on the surface or interior surface of cavities of human body having pathological changes, the characteristic of which lies in that the radiation source is a thermal radiation source capable of reaching at a temperature up to 1900-2100 K, and it has radiation guide having total reflection to the majority of radiation transmitted. This instrument is simple in structure, of high utilization of radiation energy, and of excellent effect in treating cervical erosion.

Fig. 1

## Field of Invention

The present invention relates to an optical radiation treatment instrument, and in particular, to an optical radiation treatment instrument employing the heating effect generated by radiation on the surface or interior surface of cavities of human body having pathological changes.

## Description of the Prior Art

Laser radiation can be used in the treatment for certain diseases in the cavities of human body, such as cervical erosion. For the existing laser treatment instruments, such as the LD-10B carbon dioxide laser treatment instrument produced by Ludao Company, China, the operations of which are complicated and the burning temperature of the laser on the portion of pathological changes is high, thus making the patient to feel uneasy and pain. Another deficiency of the laser treatment instrument is that the laser beam is very thin, some regions of the portion having pathological changes may not be illuminated, making it hard to provide a thorough treatment of the pathological portion of large area.

There are yet other radiation treatment instruments, however, the radiation guide means of which are of various defects, which cause relative large loss of energe in the focusing and transmission of radiation, thus the power of radiation source should be raised, and at the same time cooler is needed on the exterior of the radiation guide , in order to prevent the tissue of human body exposed to the radiation guide from being burned.

## Object of the Invention

Therefore , one of the objects of the present invention is to provide other radiation source in stead of laser in a radiation treatment instrument , producing heating effect on the pathologically changed portion and making the structure of the radiation source simple . Another object of the present invention is to provide radiation beam having thicker diameters than that (less than or equal to 2mm) of laser beams to illuminate the whole portion of pathological changes .

It is yet another object of the present invention to provide radiation beams of predetermined wave length, which reach a relatively low temperature as compared with that reached by laser, when they illuminate pathologically changed portion and normal tissues of the human body. It is a further object of the present invention to provide a radiation guide to minimize the transmission loss of the treating radiation in the radiation guide.

It is a further object of the present invention to provide reflecting and focusing means of radiation on the basis of optics theory to maximize the utilization of the energy emitted by the radiation source.

## Summary of the Invention

The present invention relates to an optical radiation treatment instrument, and in particular, to an optical radiation treatment instrument producing heating effect on the surface or interior surface of cavities of human body having pathological changes by the use of radiation, which comprises a housing, radiation source, with the reflecting mirror positioned behind the radiation source, characterized in that said radiation source is a thermal radiation source operated at temperature of 1900-2100 K, and a radiation guide provided in front of said radiation source and extending beyond said housing, which generates total reflection of major parts of the guided radiation.

The wave length of radiation emitted by radiation source having the temperature of 1900-2100 K, such as the radiation emitted by halogen tungsten lamp, is generally between 0.4-5 um, which can solidly the protein of the pathalogical cells being radiated, and cause the necrosis of the cell tissue having pathological changes.

Radiation guide, such as infrared-transmitting quartz glass rod, light-pipe, optical fibre cable and the like, are able to transmit the majority of the radiation emitted in front of or reflected by the mirror to the front of the radiation source within the radiation guide by total reflection, due to the optically thinner medium or materials with low refractivity in the surroundings, finally, the majority of the radiation emitted by the radiation source can be focused to illuminate the portion having pathological changes, the nearer the radiation guide from the radiation source, the more the radiation received.

In case of the radiation guide being infrared-transmitting quartz glass rod, the infrared radiation absorbed by the transmitting material of the rod per se are very little, so as the radiation of the radiation source can be utilized in greater efficiency.

In addition, two spherical or aspherical convex-plane cylindrical lenses can be disposed bewteen the radiation guide and the radiation source with their convex surface oppositing each other, so that the radiation emmited and reflected thereon can be concentrated into relatively uniformly distributed approximately parallel beams to make the radiation arrived at incident end of the radiation guide more uniformly concentrated, to further reduce the transmitting loss of radiation. These additional lenses are adaptable to all cases where the radiation guide is an optical fibre cable, light-pipe, or infrared transmitting quartz glass rod.

A sheath may be provided surrounding the radiation guide and some supports may be provided to fix the guide inside the sheath to form a gap between the sheath and the radiation guide, which is filled with an optically thinner medium, preferably air.

An imaging optical fibre may be further provided in parallel with the radiation guide, so as the radiated portion and condition of treatment can be observed during the treating procedure. Where the radiation guide is an optical fibre cable, at least one imaging optical fibre can be deposed in the optical fibre cable. In order to obtain clearer and larger scope observation, it is preferable to dispose an endoscope which can be mounted on the end of the imaging optical fibre.

The surface of the mirror can be plated with metal. Since different metals have different reflectivity for radiations of different frequencies, as to the radiation source employed by the present invention, that is, the great majority of radiations emitted are of the wave length 0.4-5 um, it is preferable to be plated with copper.

Cooling channels are preferably provided in the housing to cool the housing and the mirror.

The optical radiation treatment instrument of the present invention, as compared with the prior art optical radiation treatment instrument, has the following advantages:

1. A radiation source of simple structure has relatively low temperature for treatment of portions having pathological changes, large area of illumination and desirable treating effects has been employed.

2. Radiation guide and auxiliary means of minimum transmitting loss are used to achieve high efficiency and low heat loss of radiation.

The clinic practice, by using the optical radiation treatment instrument according to present invention, has showed the satisfactory result to patients who were various ages and had the various extent of cervical erosion. The statistics, to 500 cases of clinic treatment, displayed that the efficacious rate was near 100%, curative rate was 98% when the patients undergoed one time of treatment. When patients were followed up after three months, patients' pathological changes were no recurrence, elasticity of patients' pathological change portions regained to normal, and the surface of patient s'cerves returned to smooth and no obvious cicatrix.

Brief Description of the Drawings

Fig. 1 is a schematic diagram showing the structure of the optical radiation treatment instrument of embodiment 1;

Fig. 2 is a schematic diagram showing the structure of a radiation guide means having the gap between the radiation guide and the sheath filled with optically thinner medium;

Fig. 3 is a schematic diagram showing the end of the radiation guide of Fig. 2 being fabricated slantwise;

Fig. 4 is a schematic diagram showing the structure of an optical radiation treatment instrument disposed with a pair of convex-plane lenses;

Fig. 5 is a schematic diagram showing a cross-section of the radiation guide of the kind of optical fibre cable; and

Fig. 6 is a schematic diagram showing a cross-section of the radiation guide of the kind of light-pipe .

Detailed Description of the Preferred Embodiments

Embodiment 1:

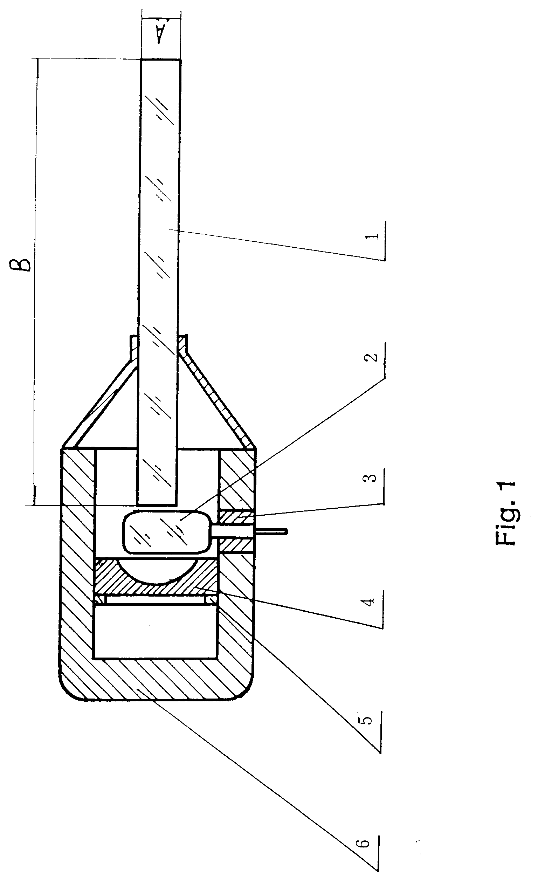

Referring to Fig. 1, an optical radiation treatment instrument comprised a housing 6, mirror 4, iodine tungsten lamp 2, and infrared transmitting quartz glass rod 1. Said housing was a cylindrical alumimum shell having a diameter of 50 mm and length of 60 mm, as well as a cooling water channel 5 behind the mirror. The concave surface of said mirror was plated with a copper coating of 0.2 mm in thickness. In the proximity of the front focus of the concave surface of said mirror, there was provided an iodine tungsten lamp of 55 volts and 500 watts. In the front of the lamp, there was mounted an infrared transmitting quartz glass rod ( Jinzhou Quartz Glass Factory, China) closing to the lamp at extending outside the housing, the diameter A of which was approximately 10 mm, and the length B of which was approximately 100 mm. The proximal end of said rod was about 1 mm from the lamp.

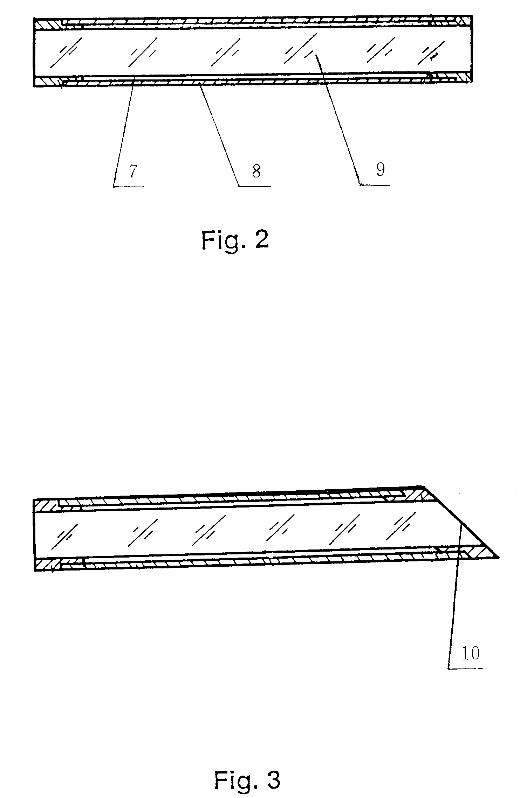

As shown in Fig. 2, a sheath surrounding said rod was provided in said instrument of Fig. 1, there was a gap 7 between the sheath 8 and the rod body 9.

As shown in Fig. 3, the emitting end 10 of said rod of the instrument of Fig. 1 was fabricated slantwise with an angle of 45.

Embodiment 2:

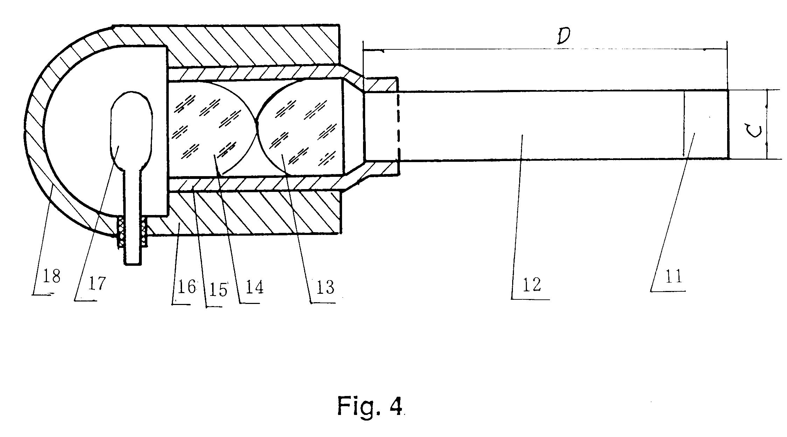

Referring to Fig. 4, an optical radiation treatment instrument comprised a housing 16, iodine tungsten lamp 17 of power 150 watts a mirror deposited on the inner surface of a section 18 of said housing, aspherical convex-plane cylindrical lenses 13 and 14, and optical fibre cable 12. Two aspherical convex-plane cylindrical lenses were provided in the inner cylinder 15 in front of the radiation source, having their convex surface oppositely contacted with each other, and the distance between said optical fibre cable and the distal convex-plane lens was about 3 mm. The emitting end of said optical fibre cable was provided with a filter 11. The diameter C of said optical fibre cable was approximately 10 mm, and the length of which was approximately 150 mm.

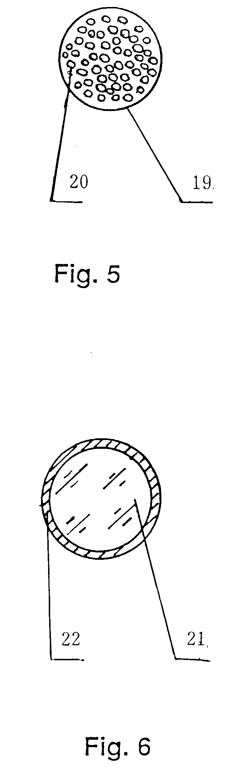

Fig. 5 is a schematic diagram of a cross-section of the optical fibre cable. The sheath 19 was made of rigid or flexible material. A plurality of optical fibres was be placed inside the sheath.

Light-pipe was also used as the radiation guide of the present embodiment, a schematic diagram of a cross-section of the light-pipe shown in Fig. 6, wherein, 21 indicated an infrared transmitting quartz glass rod, while 22 indicated a metallic pipe having its inner surface plated with copper.

**Claims**

1. An optical radiation treatment instrument, especially an optical radiation treatment instrument employing the heating effect generated by radiation on the surface or interior surface cavities of human body having pathological changes, comprising a housing, radiation source and a mirror housed in said housing with the mirror at the rear of the radiation source, characterized in that:

   a) said radiation source is a thermal radiation source having a temperature up to 1900-2100 K;

   b) a radiation guide mounted in front of radiation source extends beyond said housing and provides full reflection for the majority of radiation transmitted.

2. An optical radiation treatment instrument according to claim 1, characterized in that said radiation source is a halogen tungsten lamp.

3. An optical radiation treatment instrument according to claim 1 or 2, characterized in that said radiation guide is an infrared transmitting quartz glass rod

4. An optical radiation treatment instrument according to claim 1 or 2, characterized in that said radiation guide is an optical fibre cable.

5. An optical radiation treatment instrument according to claim 1 or 2, characterized in that said radiation guide is a light-pipe.

6. An optical radiation treatment instrument according to claim 1, characterized in that two spherical or aspherical convex-plane cylindrcal lenses are further disposed between said radiation source and said radiation guide.

7. An optical radiation treatment instrument according to claim 1 or 2, characterized in that a sheath is provided surrounding said radiation guide, said radiation guide and said sheath are fixed by supports so as to form a gap between said sheath and said radiation guide, and said gap is filled with suitable optically thinner medium, preferably air.

8. An optical radiation treatment instrument according to claim 1 or 2, characterized in that the emitting end surface of said radiation guide is a slanting surface.

9. An optical radiation treatmnet instrument according to claim 1 or 2, characterized in that said radiation guide further comprises an imaging optical fibre having an endoscope provided on one end thereof.

10. An optical radiation treatment instrument according to claim 1 or 2, characterized in that a cooling means is provided on said housing.

11. An optical radiation treatment instrument especially an optical radiation treatment instrument employing the heating effect generated by radiation on the surface or interior surface of cavities of human body having pathological changes, comprising a housing, radiation source and a mirror housed in said housing with the mirror at the rear of the radiation source, and radiation guide disposed in the front of the radiation source and extending beyond the housing, characterized in that:

    a) said radiation source is a halogen tungsten lamp;

    b) said radiation guide is an infrared transmitting quartz glass rod, and

    c) a sheath is provided surrounding said rod, and the gap between said sheath and said quartz glass rod is filled with an optically thinner medium.

12. An optical radiation treatment instrument , especially an optical radiation treatment instrument employing the heating effect generatedby radiation on the surface or interior surface cavities of human body having pathological changes, comprising a housing, radiation source and a mirror housed in said housing with the mirror at the rear of the radiation source, and radiation guide disposed in the front of the radiation source and extending beyond said housing, characterized in that:

a) said radiation source is a halogen tungsten lamp; and

b) two spherical or aspherical convex-plane cylindrical lenses are disposed between said radiation source and said radiation guide and have their convex surface oppositing each other.

EP 0 495 443 A1

Fig. 1

Fig. 2

Fig. 3

Fig. 4

Fig. 5

Fig. 6

## DOCUMENTS CONSIDERED TO BE RELEVANT

EP 92100477.6

| Category | Citation of document with indication, where appropriate, of relevant passages | Relevant to claim | CLASSIFICATION OF THE APPLICATION (Int. Cl.5) |
|---|---|---|---|
| X | DE - A - 2 717 421 (G. NATH) * Fig. 1; claims 1-3; page 8, last line; page 10, last paragraph - page 11, line 7; page 12, last paragraph * | 1-3 | A 61 N 5/06 A 61 F 7/12 A 61 B 17/36 |
| Y | | 4,5,7, 10 | |
| A | | 11,12 | |
| Y | DE - A - 3 803 763 (B. VUGHT) * Totality * | 4 | |
| A | | 1,6 | |
| Y | US - A - 4 805 987 (K. LAAKMANN et al.) * Fig. 1; abstract * | 5 | |
| Y | DE - A - 2 511 037 (G. NATH) * Fig. 2; page 7, last para- graph - page 8, line 6 * | 5 | TECHNICAL FIELDS SEARCHED (Int. Cl.5) |
| A | | 1,2 | A 61 B |
| X | DE - A - 2 846 471 (G. NATH) * Totality; especially fig. 1-3; page 8, 7. paragraph - page 10, first paragraph * | 11 | A 61 F A 61 N G 02 B |
| Y | | 7 | |
| A | | 1-3,10 | |
| X | US - A - 1 800 277 (E.W. BOERSTLER) * Totality * | 11 | |
| Y | | 7 | |
| Y | DE - A - 2 607 249 (G. NATH) | 10 | |

The present search report has been drawn up for all claims

| Place of search | Date of completion of the search | Examiner |
|---|---|---|
| VIENNA | 28-04-1992 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                         

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | * Fig. 1; page 18, lines 13-15 * | 1-3,7 | |
| A | DE - A - 3 347 730 (U. PESCH) * Totality * | 1,2 | |

TECHNICAL FIELDS SEARCHED (Int. Cl.5)

The present search report has been drawn up for all claims

| Place of search | Date of completion of the search | Examiner |
|---|---|---|
| VIENNA | 28-04-1992 | LUDWIG |